(19) Europäisches Patentamt
European Patent Office
Office européen des brevets

(11) **EP 4 467 075 A1**

(12) **EUROPEAN PATENT APPLICATION**
published in accordance with Art. 153(4) EPC

(43) Date of publication:
**27.11.2024 Bulletin 2024/48**

(21) Application number: **22926232.4**

(22) Date of filing: **28.12.2022**

(51) International Patent Classification (IPC):
**A61B 5/1455** $^{(2006.01)}$     **A61B 5/145** $^{(2006.01)}$
**A61B 5/00** $^{(2006.01)}$

(52) Cooperative Patent Classification (CPC):
**A61B 5/00; A61B 5/145; A61B 5/1455**

(86) International application number:
**PCT/KR2022/021494**

(87) International publication number:
**WO 2023/153636 (17.08.2023 Gazette 2023/33)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **14.02.2022 KR 20220019076**

(71) Applicant: **Samsung Electronics Co., Ltd.**
**Gyeonggi-do, 16677 (KR)**

(72) Inventors:
• **KIM, Younggeol**
  **Suwon-si Gyeonggi-do 16677 (KR)**
• **SHIN, Euiseok**
  **Suwon-si Gyeonggi-do 16677 (KR)**

(74) Representative: **Appleyard Lees IP LLP**
**15 Clare Road**
**Halifax HX1 2HY (GB)**

(54) **ELECTRONIC DEVICE PROVIDING ADVANCED GLYCATION END PRODUCTS DATA OF SKIN AND METHOD FOR OPERATING SAME**

(57) According to various embodiments, an electronic device comprises: a first light source configured to emit light of a first wavelength band corresponding to UV light; a second light source configured to emit light of a second wavelength band corresponding to visible light; a sensing circuit for measuring the intensity of incident light and outputting the measured intensity of light; and a processor. The processor may be configured to: receive, from the sensing circuit, a first intensity of light corresponding to a third wavelength band at least partially different from the first wavelength band, the first intensity being measured by the sensing circuit while the first light source is operating; receive, from the sensing circuit, second intensities of light respectively corresponding to one or more wavelength bands included in the second wavelength band, the second intensities being measured by the sensing circuit while the second light source is operating; and provide advanced glycation end products data of skin on the basis of the received first intensity of light and the received one or more second intensities of light.

FIG. 5

# Description

[Technical Field]

The disclosure relates to an electronic device that provides advanced glycation end products data through skin autofluorescence and a method for operating the same.

[Background Art]

Advanced glycation end products (AGEs) are formed in the oxidation of proteins in body organs as a result of the Maillard reaction. Advanced glycation end products (AGEs) accumulated in the skin are substances that reduce functionality by combining sugar with proteins in tissues due to excessive blood sugar, resulting in decreased vascular function and diabetic diseases.

Skin autofluorescence for advanced glycation end products is a method for measuring advanced glycation end products (AGEs) in human skin using autofluorescence, which increases the possibility of diagnosing various human diseases related to diabetes.

[Detailed Description of the Invention]

[Technical Problem]

Advanced glycation end products may be measured using a spectrometer, but the spectrometer has the disadvantage of being relatively expensive and bulky. Further, advanced glycation end products may be measured using a bandpass filter for a specific wavelength band alone, but the bandpass filter is relatively expensive.

According to various embodiments, there may be provided an electronic device that provides advanced glycation end products data by non-invasively measuring the amount of skin autofluorescence and a method for operating the same. Therefore, skin autofluorescence may be measured using a downsized and inexpensive electronic device.

[Technical Solution]

According to various embodiments, an electronic device comprises a first light source configured to irradiate light of a first wavelength band corresponding to UV light, a second light source configured to irradiate light of a second wavelength band corresponding to visible light, the second wavelength band at least partially different from the first wavelength band, a sensing circuit configured to measure an intensity of incident light and output the measured intensity of light, and a processor configured to receive, from the sensing circuit, a first intensity of light corresponding to a third wavelength band at least partially different from the first wavelength band measured by the sensing circuit while the first light source operates, receive, from the sensing circuit, a second intensity of light respectively corresponding to at least one wavelength band included in the second wavelength band measured by the sensing circuit while the second light source operates, and provide advanced glycation end products (AGEs) data of skin based on the received first intensity of light and the received second intensity of at least one light.

According to various embodiments, a method for operating an electronic device comprises receiving, from a sensing circuit, a first intensity of light corresponding to a third wavelength band at least partially different from a first wavelength band measured by the sensing circuit while a first light source configured to irradiate light of the first wavelength band corresponding to UV light operates and a second intensity of light corresponding to at least one wavelength band included in a second wavelength band measured by the sensing circuit while a second light source configured to irradiate light of the second wavelength band corresponding to visible light operates and providing skin advanced glycation end products based on the received first intensity of light and second intensity of at least one light.

According to various embodiments, an electronic device comprises a sensing circuit configured to measure an intensity of incident light and outputting the measured intensity of light and a processor configured to receive, from the sensing circuit, a first intensity of light corresponding to a third wavelength band at least partially different from a first wavelength band measured by the sensing circuit while the light of the first wavelength band corresponding to UV light is irradiated, receive, from the sensing circuit, a second intensity of light respectively corresponding to at least one wavelength band included in a second wavelength band measured by the sensing circuit while light of the second wavelength band corresponding to visible light is irradiated, the second wavelength band at least partially different from the first wavelength band, and provide advanced glycation end products (AGEs) data of skin based on the received first intensity of light and the received second intensity of the at least one light.

[Advantageous Effects]

According to various embodiments, there may be provided an electronic device that provides advanced glycation end products data by accurately measuring the amount of skin autofluorescence in a non-invasive manner and a method for operating the same. Further, skin autofluorescence may be measured using a downsized and inexpensive electronic device.

[Brief Description of the Drawings]

FIG. 1 is a block diagram illustrating an electronic device according to various embodiments;

FIG. 2 is a flowchart illustrating operations of an electronic device according to various embodiments;

FIG. 3 is a wavelength-intensity graph of autofluorescence according to various embodiments;

FIG. 4 is a table for autofluorescence characteristics of an organism according to various embodiments;

FIG. 5 is a block diagram illustrating operations of an electronic device according to various embodiments;

FIG. 6 is a block diagram illustrating a state in which light of a first wavelength band is irradiated by an electronic device according to various embodiments;

FIG. 7 is a wavelength-intensity graph of light of a first wavelength band according to various embodiments;

FIG. 8 is a wavelength-intensity graph of light of a third wavelength band and RGB sensor reacting light according to various embodiments;

FIG. 9 is a block diagram illustrating a state in which light of a second wavelength band is irradiated by an electronic device according to various embodiments;

FIG. 10 is a wavelength-intensity graph of light of a second wavelength band according to various embodiments;

FIG. 11 is a wavelength-intensity graph of light corresponding to at least one wavelength band included in a second wavelength band and RGB sensor reacting light according to various embodiments;

FIG. 12 is a graph of light intensity and skin reflectance in each wavelength area according to various embodiments;

FIG. 13A is a cross-sectional view illustrating an electronic device according to various embodiments, and FIG. 13B is a bottom view illustrating an electronic device according to various embodiments;

FIG. 14 illustrates an application example of an electronic device according to an embodiment;

FIG. 15 illustrates an application example of an electronic device according to other embodiments; and

FIG. 16 is a view illustrating an electronic device in a network environment according to various embodiments.

[Mode for Carrying out the Invention]

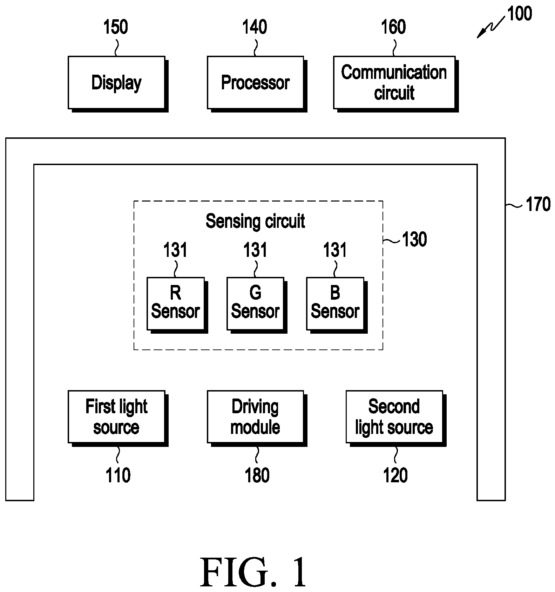

FIG. 1 is a block diagram illustrating an electronic device 100 according to various embodiments. According to various embodiments, the electronic device 100 may include at least one of a first light source 110, a second light source 120, a sensing circuit 130, a processor 140, a display 150, a communication circuit 160, a light blocking housing 170, and a driving module 180.

In an embodiment, the first light source 110 and the second light source 120 may be operated by receiving power from a power storage device such as a battery (not shown) provided in the electronic device 100 or from the outside, and may emit light of each set wavelength band during operation.

The first light source 110 may be configured to irradiate light of a first wavelength band corresponding to UV light. Specifically, the UV light may be a short wavelength band of 100 to 400 [nm], and the first wavelength band may include all or some wavelength bands of 100 to 400 [nm] corresponding to the UV light. In an embodiment, the first light source 110 may irradiate light of a first wavelength band of 315 to 400 [nm] corresponding to UV-A light, and in particular, may irradiate light of a specific wavelength area of the wavelength band of UV-A light. As is described below, the skin that absorbs light of the first wavelength band may emit light of a wavelength band different from the first wavelength band through autofluorescence.

The second light source 120 may be configured to emit light of a second wavelength band corresponding to visible light. Specifically, the visible light may be a wavelength band of 380 to 780 [nm], and the second wavelength band may include the entire wavelength band of the visible light or may include a partial wavelength band of the visible light having a specific color. In an embodiment, the second light source 120 may irradiate light evenly distributed over the second wavelength band corresponding to visible light. In another embodiment, the second light source 120 may emit light of the second wavelength band corresponding to some wavelength bands of visible light.

The first light source 110 and/or the second light source 120 according to various embodiments may be an electric light emitting light using electrical energy, and may include at least one of an incandescent light, a discharge light, or a light emitting diode (LED), but is not limited thereto. For example, the first light source 110 and/or the second light source 120 may be implemented as a single light source or may be implemented to include a plurality of sub light sources.

The sensing circuit 130 may measure the intensity of incident light. The sensing circuit 130 may include an optical sensor that senses an optical signal of visible light, ultraviolet light, and/or infrared light that may be classified as an optical area among electromagnetic waves and converts the optical signal into an electrical signal. In an embodiment, the sensing circuit 130 may include a photodiode that converts light energy into electrical energy. The sensing circuit 130 may output the measured intensity of light as an electrical signal.

In an embodiment, the sensing circuit 130 may include an RGB sensor 131. The RGB sensor may distinguish light respectively corresponding to the red wavelength band, the green wavelength band, and the blue wavelength band and measure the intensity of each light. In an embodiment, the RGB sensor 131 may distinguish and measure the light intensities of wavelength bands respectively included in a preset red wavelength band (e.g., 630 to 780 [nm]), a preset green wavelength band (e.g., 510 to 540 [nm]), and a preset blue wavelength band (e.g., 450 to 480 [nm]). For example, the RGB sensor 131 may measure the intensity of the wavelength band incident through each of a red, green, and blue color filter array (CFA). In another embodiment, some wavelength bands of the preset red wavelength band, the green wavelength band, and the blue wavelength band may overlap each other.

The RGB sensor 131 may be composed of a plurality of pixels arranged in an array form for measuring the intensity of light incident on the plurality of areas, respectively, and may measure the intensity of light incident through the plurality of areas. In an embodiment, in the RGB sensor 131, the color filter array CFA may be formed in a plurality of areas, e.g., may be disposed in a Bayer pattern. The RGB sensor 131 may include an R sensor for measuring the intensity of light in the red wavelength area, a G sensor for measuring the intensity of light in the green wavelength area, and a B sensor for measuring the intensity of light in the blue wavelength area.

In general, the RGB sensor 131 arranged in an array form has a structure in which a plurality of pixels are arranged in a Bayer pattern, and each pixel has a structure in which only one color is incident, and thus goes through image processing in which each color is mixed through an interpolation process (e.g., Demosaic). However, the electronic device according to various embodiments may be configured to receive at least one of the intensity of light of the blue wavelength band, the intensity of light of the green wavelength band, or the intensity of light of the red wavelength band, measured by the RGB sensor, in a state of raw data state (RAW data) that is not subjected to image processing. In various embodiments, the electronic device may be configured not to perform an interpolation process when the advanced glycation end products data is identified, and may be configured to perform an interpolation process when a general image is captured through the sensing circuit 130.

For example, the RGB sensor 131 outputs data in a state of raw data that is not subjected to image processing such as gamma or jpeg. Here, the light intensity data output by the RGB sensor 131 becomes a 2D image having a value (component) for each of R, G, and B, and the electronic device according to various embodiments extracts a representative value of the data measured by the plurality of pixels. For example, an average value, a median value, or a mode value may be used to calculate the representative value.

The electronic device according to various embodiments may extract representative values for different areas (pixels) because differences may occur for each skin area, and then may calculate a final representative value again from the extracted representative values.

According to an embodiment, in case the intensity of light is saturated in some of the plurality of pixels due to the highlight of the light reflected from the skin, a polarizing filter may be used, or data of the corresponding pixel may be excluded when the representative value is extracted.

The processing module 140 may include one or more of a central processing unit (CPU), an application processor (AP), or a communication processor (CP). The processor 140 may perform control on at least one of the other components of the electronic device 100, and/or perform an operation or data processing relating to communication. The processor 140 may execute, e.g., software to control at least one other component (e.g., a hardware or software component) of the electronic device 100 connected with the processor 140 and may process or compute various data. According to an embodiment, as at least part of the data processing or computation, the processor 140 may load a command or data received from another component (e.g., the sensing circuit 130 or communication circuit 160) onto a volatile memory (not shown), process the command or the data stored in the volatile memory, and store resulting data in a non-volatile memory. The memory (not shown) may store commands or data related to at least one other component of the electronic device 100.

The display 150 is an output device that provides visual information to the outside (e.g., the user) of the electronic device 100 and may include, e.g., a liquid crystal display (LCD), a light emitting diode (LED) display, an organic light emitting diode (OLED) display, or a microelectromechanical systems (MEMS) display, or an electronic paper display. The display 150 may display, e.g., various contents (e.g., text, images, videos, icons, or symbols) to the user. The display 150 may include a touchscreen and may receive, e.g., a touch, gesture, proximity, drag, swipe, or hovering input using an electronic pen or a body portion of the user. The display 150 may provide advanced glycation end products data of skin, as visual information, through a separate control circuit.

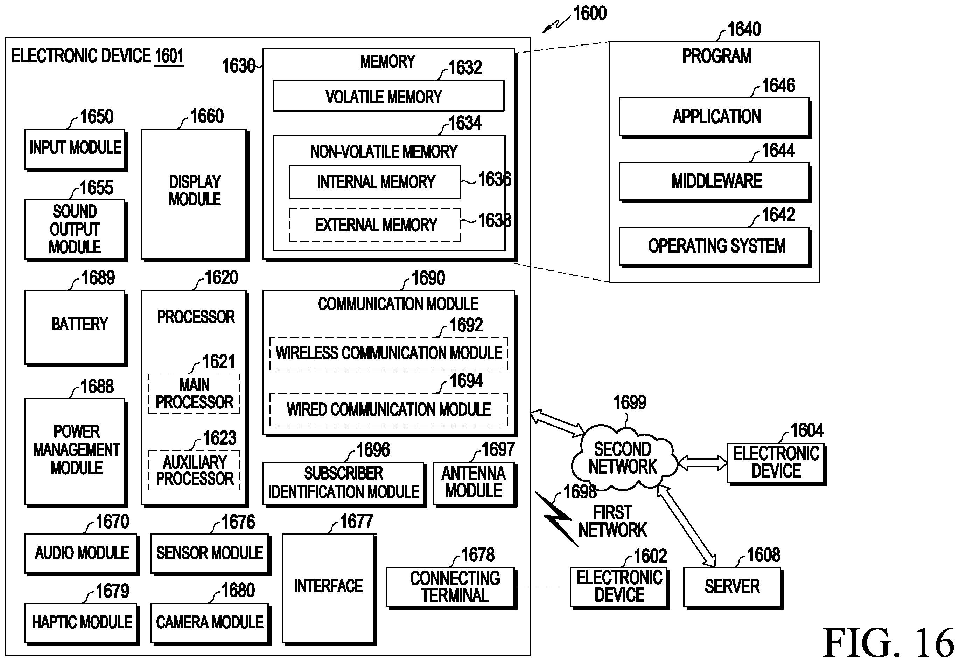

The communication circuit 160 may include a wireless communication module (e.g., a cellular communication module, a wireless-fidelity (Wi-Fi) communication module, a Bluetooth communication module, a near-field communication (NFC) communication module, or a global navigation satellite system (GNSS) communication module) or a wired communication module (e.g., a local area network (LAN) communication module or a power line communication (PLC) module). The communication circuit 160 including a corresponding communication module of the wired communication module or the wireless communication module may communicate with an external electronic device 100 (e.g., a portable terminal) through a first network (e.g., the first network 1698 of FIG. 16) or a second network (e.g., the second network 1699 of FIG. 16). The communication circuit 160 may include one or more communication processors 140 that are operable independently from the processor 140 and supports a direct (e.g., wired) communication or a wireless communication.

Additionally, the communication circuit 160 may be connected to an antenna circuit (not shown) capable of transmitting a signal or power to or receiving a signal or power from another electronic device 100 (e.g., a portable terminal). The antenna circuit may include one antenna including a conductor formed on a substrate (e.g., a PCB) or a radiator formed of a conductive pattern. According to an embodiment, the antenna circuit may include a plurality of antennas. In this case, at least one antenna appropriate for a communication scheme used in a communication network, such as the first network (e.g., the first network 1698 of FIG. 16) or the second network (e.g., the second network 1699 of FIG. 16), may be selected from the plurality of antennas by, e.g., the communication circuit 160. The signal or the power may then be transmitted or received between the communication circuit 160 and another electronic device 100 via the selected at least one antenna. According to an embodiment, other parts (e.g., radio frequency integrated circuit (RFIC)) than the radiator may be further formed as part of the antenna circuit.

The light blocking housing 170 may be formed so that one surface of the light blocking housing 170 that is in tight contact with the skin is open or transparent to transmit light, and the periphery of the one surface of the light blocking housing 170 may be formed of an opaque material that blocks light. The light blocking housing 170 may block light from entering from the outside to the inside or light from the inside from being emitted to the outside.

In an embodiment, the first light source 110, the second light source 120, the sensing circuit 130 (e.g., the RGB sensor 131), and the driving module 180 may be surrounded by the light blocking housing 170, and in a state where one surface of the light blocking housing 170 is in tight contact with the skin, the light blocking housing 170 and the skin may block the inflow of light from the outside or the leakage of light to the outside.

The driving module 180 may drive movement of the optical lens and/or opening and closing of the shutter, or may drive to control the focus and/or an iris. In an embodiment, the driving module 180 may include a shutter that is closed to block light incident on an optical sensor (e.g., the RGB sensor 131) included in the sensing circuit 130 or opened to allow light incident on the optical sensor to pass therethrough. Considering the performance, sensitivity, and/or reaction time of the optical sensor, the opening/closing of the shutter may be adjusted to an opening time when the intensity of light measured by the optical sensor is saturated or which is not insufficient for the optical sensor to sense the intensity of light.

According to various embodiments, the electronic device 100 may further include various circuits depending on the form in which it is provided. There are many variations according to the convergence trend of digital devices, so it is not possible to list them all, but components equivalent to the above-mentioned components may be further included in the electronic device 100. Further, it is apparent that in the electronic device 100 according to an embodiment, specific components may be excluded from the above components or replaced with other components according to the form in which it is provided. This will be easily understood by those of ordinary skill in the art.

According to various embodiments, operations described in the disclosure may be operations performed by the processor 140 of the electronic device 100 unless otherwise specified.

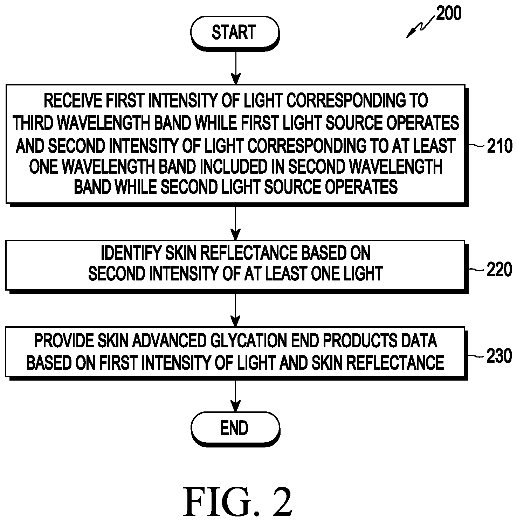

FIG. 2 is a flowchart 200 illustrating operations of an electronic device according to various embodiments. According to various embodiments, in operation 210, an electronic device (e.g., the processor 140 of FIG. 1) may receive, from a sensing circuit, a first intensity of light corresponding to a third wavelength band at least partially different from a first wavelength band measured by the sensing circuit (e.g., the sensing circuit 130 of FIG. 1) while a first light source (e.g., the first light source 110 of FIG. 1) configured to irradiate light of the first wavelength band corresponding to UV light operates and a second intensity of at least one light corresponding to at least one wavelength band included in a second wavelength band measured by the sensing circuit while a second light source (e.g., the second light source 120 of FIG. 1) configured to irradiate light of the second wavelength band corresponding to visible light operates.

The sensing circuit (e.g., the sensing circuit 130 of FIG. 1) may measure the first intensity of light corresponding to the third wavelength band at least partially different from the first wavelength band while the first light source irradiates light of the first wavelength band. When light of the first wavelength band of the first light source is irradiated to the human body, light corresponding to the third wavelength band may be provided from the human body, which is described below. The sensing circuit may measure the second intensity of the light corresponding to at least one wavelength band included in the second wavelength band while the second light source irradiates the light of the second wavelength band. When light of the second wavelength band of the second light source is irradiated to the human body, light corresponding to at least one wavelength band may be provided from the human body, which is described below.

According to various embodiments, in operation 210, the electronic device may sequentially perform the operation of receiving the first intensity of light corresponding to the third wavelength band at least partially different from the first wavelength band measured by the sensing circuit while the first light source operates, and the operation of receiving the second intensity of at least one light corresponding to at least one wavelength band included in the second wavelength band measured by the sensing circuit while the second light source operates. According to an embodiment, after receiving the first intensity of light corresponding to the third wavelength band, the second intensity of at least one light corresponding to at least one wavelength band included in the second wavelength band may be received, or after receiving the second intensity of at least one light corresponding to at least one wavelength band included in the second wavelength band, the first intensity of light corresponding to the third wavelength band may be received. Meanwhile, depending on implementation, reception intensity measurement of both lights may be performed simultaneously for at least a partial period of time.

According to various embodiments, the electronic device (e.g., the processor 140 of FIG. 1) may identify skin reflectance based on the received second intensity of the at least one light in operation 220.

The electronic device may receive the second intensity of the light corresponding to at least one wavelength band included in the second wavelength band measured by the sensing circuit while the second light source irradiates the light of the second wavelength band, and identify the skin reflectance based on the received second intensity of the at least one light.

According to various embodiments, in operation 230, the electronic device (e.g., the processor 140 of FIG. 1) may provide advanced glycation end products data of the skin, based on the first intensity of the received light and the skin reflectance.

Specifically, the electronic device may identify the amount of autofluorescence of the skin based on the first intensity of the light received from the sensing circuit, and identify the skin reflectance based on the second intensity of the at least one light received from the sensing circuit. The electronic device may apply the skin reflectance to the identified autofluorescence amount of the skin to finally identify the advanced glycation end products data of the skin. For example, the electronic device may identify the advanced glycation end products data of the skin based on the first intensity of the light and the second intensity of the at least one light received from the sensing circuit.

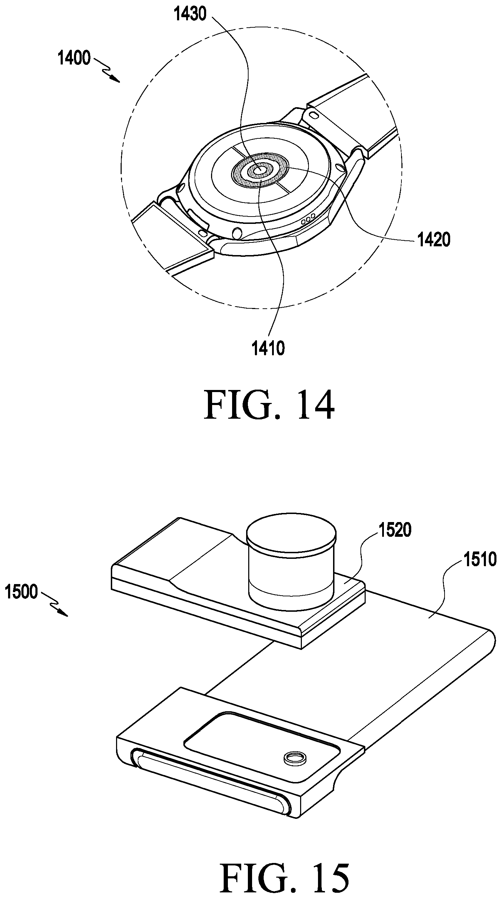

In an embodiment, the electronic device may output the identified advanced glycation end products data of the skin to the display (the display 150 of FIG. 1) as visual information (or output through another type of output device), or may provide the identified advanced glycation end products data to the communication circuit (the communication circuit 160 of FIG. 1) as data transmitted to an external device (e.g., the external device 1510 of FIG. 15).

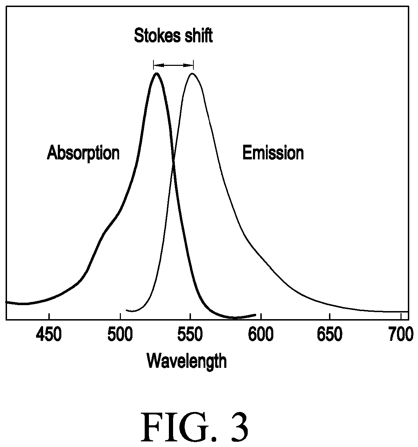

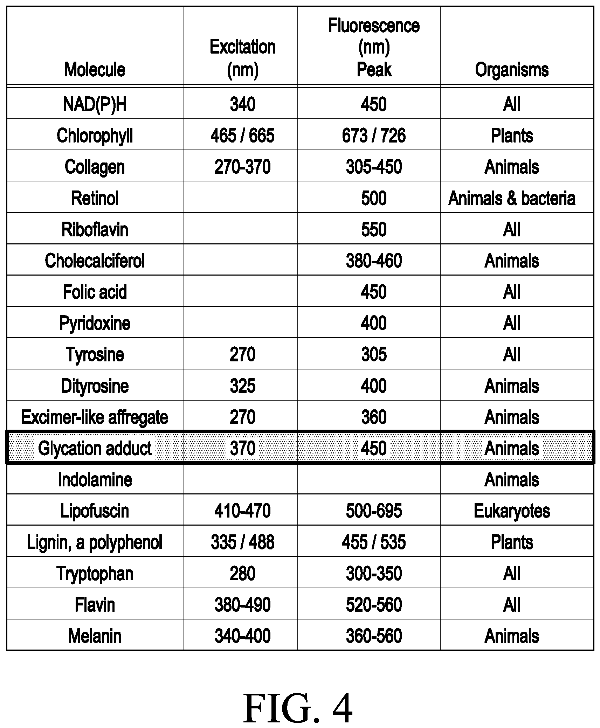

FIG. 3 is a wavelength-intensity graph of autofluorescence according to various embodiments, and FIG. 4 is a table illustrating autofluorescence characteristics of an organism according to various embodiments.

Autofluorescence is a phenomenon in which when a molecular structure absorbs UV light or visible light, an energy state becomes an excited state of electrons from a ground state, and as the energy state transitions from the excited state to another state (e.g., a state having a relatively lower energy level than the excited state), energy is emitted to emit light of a wavelength band relatively longer than the emitted light.

Each of the various molecules included in the organism has different autofluorescence characteristics. Advanced glycation end products (glycation adduct) has the characteristic of radiating light of a wavelength band (blue wavelength band of visible light) having the highest intensity of light at 450 [nm] when absorbing light of a wavelength band (corresponding to UV-A light) having the highest intensity of light at 370 [nm].

An electronic device (e.g., the electronic device 100 of FIG. 1) according to various embodiments may irradiate light of a first wavelength band that excites autofluorescence of the skin to the skin, and the first wavelength band may be a band including a wavelength of 370 [nm],

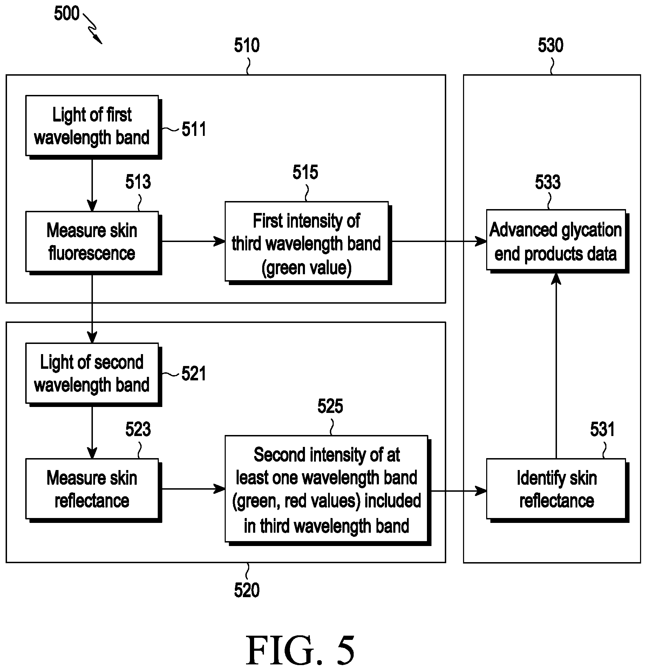

FIG. 5 is a block diagram 500 illustrating operations of an electronic device (e.g., the electronic device 100 of FIG. 1) according to various embodiments.

The electronic device according to various embodiments may perform an operation of measuring the amount of fluorescence of the skin in operation 510. Specifically, in operation 511, the electronic device may irradiate the light of the first wavelength band to the skin through a first light source (e.g., the first light source 110 of FIG. 1) configured to irradiate the light of the first wavelength band corresponding to the UV light. In operation 511, the first light source may irradiate the light of the first wavelength band at an intensity of light preset not to be harmful to the human body when the light is irradiated for a short time. In an embodiment, the electronic device may calculate a coefficient between the intensity of the irradiated light and the intensity of the light measured by reflection by measuring the intensity of the first wavelength band reflected from a standard reflective object in a state in which the first light source irradiates the light at a predetermined intensity of light to the standard reflective object whose reflectance was known.

Thereafter, in operation 513, the electronic device may measure skin fluorescence through a sensing circuit (e.g., the sensing circuit 130 or the RGB sensor 131 of FIG. 1). According to an embodiment, the electronic device may measure the amount of fluorescence of the skin while the light of the first wavelength band is irradiated.

In operation 515, the electronic device may receive the first intensity of light corresponding to the third wavelength band measured by the sensing circuit. Here, the third wavelength band is at least partially different from the first wavelength band corresponding to UV light, and may be, e.g., a wavelength band corresponding to visible light. In an embodiment, the third wavelength band may be set to a green wavelength band of visible light rather than a wavelength band (a blue wavelength band of visible light) having the highest intensity of light, as is described below. The sensing circuit may measure the intensity AF_UV of light corresponding to the third wavelength band, and the electronic device (e.g., the processor 140 of FIG. 1) may receive the intensity AF_UV of light measured from the sensing circuit as the first intensity of light.

According to an embodiment, the electronic device may calculate the coefficient by floating between 0 and 1 (the measured intensity AF_UV of the light / the intensity of the irradiated light) or normalizing to a unit value based on 0 to 100 or 0 to 255, 0 to 65535, for the intensity AF_UV of the light corresponding to the third wavelength band. Accordingly, the production yield deviation between the first light source and/or the second light source and the sensing circuit (e.g., the sensing circuit 130 or the RGB sensor 131 of FIG. 1) may be supplemented.

In operation 520, the electronic device according to various embodiments may measure skin reflectance. Here, it is shown that operation 520 is performed after operation 510, but the disclosure is not limited thereto, and operation 520 for measuring skin reflectance may be performed before operation 510.

Specifically, in operation 521, the electronic device may irradiate light of the second wavelength band to the skin through a second light source (e.g., the second light source 120 of FIG. 1) configured to irradiate light of the second wavelength band corresponding to visible light. In operation 521, the second light source may irradiate light of the second wavelength band with a preset intensity of light. According to an embodiment, the electronic device may calculate the coefficient between the intensity of the irradiated light and the intensity of the light measured by the reflection by measuring the intensity of the light of the second wavelength band reflected from the standard reflective object in a state in which the second light source irradiates the light to the standard reflective object whose reflectance is known, with a predetermined intensity of the light.

Thereafter, in operation 523, the electronic device may measure skin reflectance through a sensing circuit (e.g., the sensing circuit 130 or the RGB sensor 131 of FIG. 1).

According to an embodiment, in operation 525, the electronic device may receive the second intensity of light corresponding to at least one wavelength band included in the second wavelength band measured by the sensing circuit. Here, the at least one light is the wavelength band included in the second wavelength band corresponding to visible light, and may be a partial wavelength band or the entire wavelength band of the second wavelength band. The sensing circuit may measure the intensity AF_VR of light corresponding to a partial wavelength band or the entire wavelength band of the second wavelength band, and the electronic device (e.g., the processor 140 of FIG. 1) may receive the intensity AF_VR of light measured from the sensing circuit as the second intensity of light.

In operation 530, the electronic device according to various embodiments may provide advanced glycation end products data. Specifically, in operation 531, the electronic device may identify skin reflectance based on the second intensity of at least one wavelength band included in the second wavelength band obtained in operation 520. According to an embodiment, the electronic device may identify the skin reflectance based on the intensity of light of the second wavelength band irradiated from the second light source and the second intensity of at least one wavelength band included in the second wavelength band with the preset intensity of light. For example, the electronic device may identify the second intensity of at least one wavelength band included in the second wavelength band measured by the sensing circuit and received, relative to the intensity of light of the second wavelength band irradiated from the second light source, as a skin reflectance. According to an embodiment, the identified skin reflectance may be calculated by floating between 0 and 1 using the equation (x - Min / (Max + Min)) or normalizing to a unit value based on 0 to 100 or 0 to 255, 0 to 65535.

In operation 532, the electronic device according to various embodiments may provide advanced glycation end products data based on the first intensity of light of the third wavelength band obtained in operation 510, and the skin reflectance identified in operation 531. According to an embodiment, the advanced glycation end products data may be identified by subtracting the intensity of the reflected light according to the identified skin reflectance from the first intensity of the light of the third wavelength band. For example, the advanced glycation end products data may be identified as a difference between coefficients scaled by floating or normalizing in each step.

In another embodiment, without identifying the skin reflectance, the advanced glycation end products data may be identified by subtracting the second intensity of at least one wavelength band included in the second wavelength band obtained in operation 520 from the first intensity of light of the third wavelength band obtained in operation 510. Here, the electronic device may apply appropriate scaling to the second intensity of at least one wavelength band included in the second wavelength band and subtract it from the first intensity of the third wavelength band light.

Thereafter, the electronic device may identify the health condition of the user based on the identified advanced glycation end products data. Autofluorescence of the skin may be correlated with ages (aging) and/or glycated hemoglobin (HbA1c). By using the measurement data according to the clinical trial, each coefficient meeting regression of indexes of glycated hemoglobin (HbA1c) and ages may be generated. For example, by analyzing the correlation between ages and glycated hemoglobin (HbA1c) and autofluorescence through regression analysis according to clinical trials, health diagnosis according to autofluorescence data may be possible.

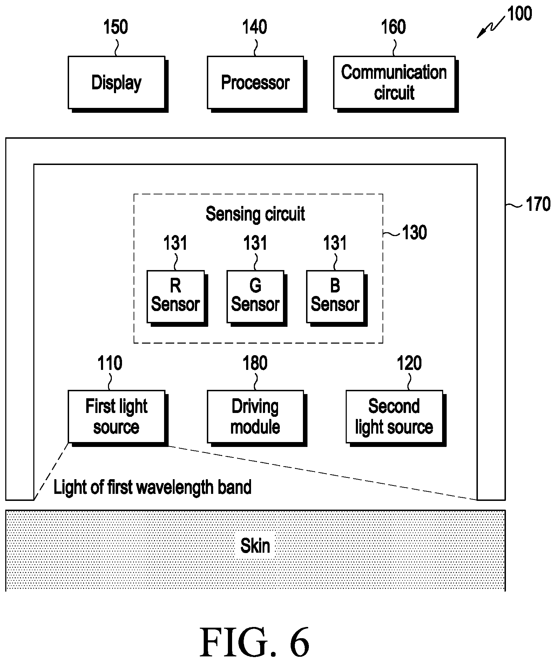

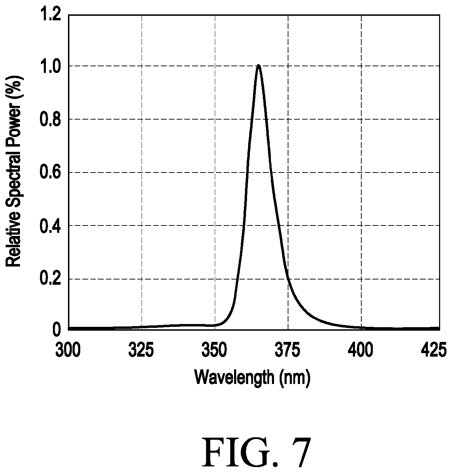

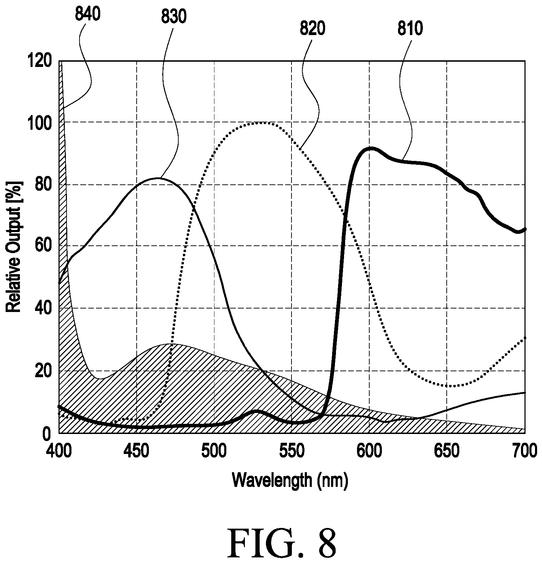

FIG. 6 is a block diagram illustrating a state in which light of a first wavelength band is irradiated by an electronic device according to various embodiments, FIG. 7 is a wavelength-intensity graph of light of a first wavelength band according to various embodiments, and FIG. 8 is a wavelength-intensity graph of light of a third wavelength band and RGB sensor reacting light according to various embodiments.

The electronic device according to various embodiments may operate the first light source to irradiate light of the first wavelength band corresponding to UV light to the skin.

In an embodiment, as illustrated in FIG. 7, the first wavelength band may be a wavelength band corresponding to a wavelength band of UV-A light. For example, the wavelength band of UV-A light, which is the first wavelength band, may be 315 to 400 [nm]. Further, the first wavelength band may have a relatively narrow wavelength range with respect to a specific wavelength. For example, the wavelength having the highest intensity of light of the first wavelength band may be 365 [nm].

Additionally, the first light source may be preset to irradiate light with an intensity that is not harmful even when irradiated to the human body, and may be configured to operate during a time when the first intensity of the light measured by the sensing circuit 130 or the RGB sensor 131 is saturated or a result value that is not insufficient is generated.

As illustrated in FIG. 8, if the light of the first wavelength band is irradiated to the skin, the skin may irradiate the light 840 of the third wavelength band at least partially different from the first wavelength band according to the autofluorescence phenomenon. In an embodiment, as UV-The light of the first wavelength band having a wavelength of 365 [nm] having the highest intensity of light is irradiated to the skin, the intensity of light according to reflection and fluorescence is shown in graph 840 (FIG. 8). Here, the light around 400 [nm] may be due to reflection of light having an ambient wavelength of 365 [nm], and the light of 450 [nm] or more may be due to fluorescence. Depending on the human skin, the shape of the curve may be the same, but the height (light intensity) of the curve may be partially different.

As illustrated in FIG. 8, the RGB sensor may measure the intensity of each of the light 810, 820, and 830 at least partially corresponding to different wavelength bands. Specifically, the RGB sensor may include an R sensor for measuring the intensity of the light 810 of the red wavelength area, a G sensor for measuring the intensity of the light 820 of the green wavelength area, and a B sensor for measuring the intensity of the light 830 of the blue wavelength area. As illustrated in FIG. 8, the RGB sensor may measure the intensity of light corresponding to the red wavelength area 810, the green wavelength area 820, and the blue wavelength area 830 in the R sensor, the G sensor, and the B sensor having response curves in different wavelength areas.

The sensing circuit may measure the first intensity of light of the third wavelength band. The third wavelength band may be set as a partial wavelength band of visible light set to be larger than a first boundary wavelength between visible light and UV and smaller than a second boundary wavelength between visible light and infrared light.

In an embodiment, the third wavelength band may correspond to a green wavelength band among visible light wavelength bands, and the electronic device may receive the intensity of light corresponding to the green wavelength band from the sensing circuit as the first intensity of light while the first light source operates. The light of the green wavelength band measured by the G sensor included in the RGB sensor includes a significant portion of skin autofluorescence having a wavelength near 450 [nm], and at the same time has low reactivity to the light of the first wavelength band irradiated from the first light source and reflected. Accordingly, light of the first wavelength band reflected from the skin may be rejected, and the intensity of light according to autofluorescence of the skin may be measured.

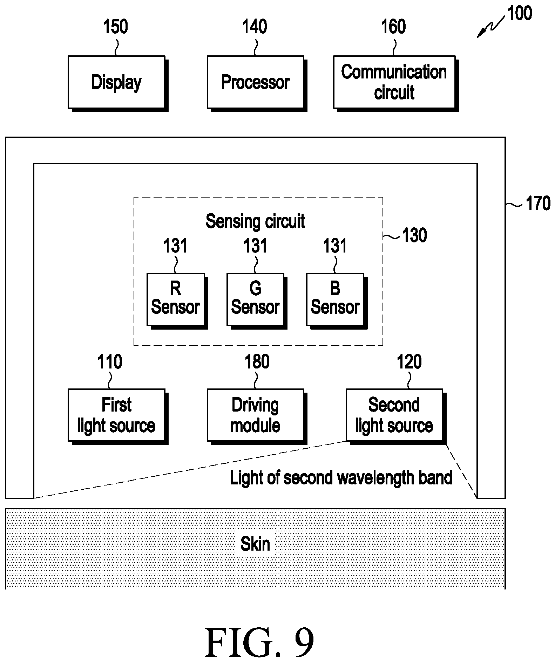

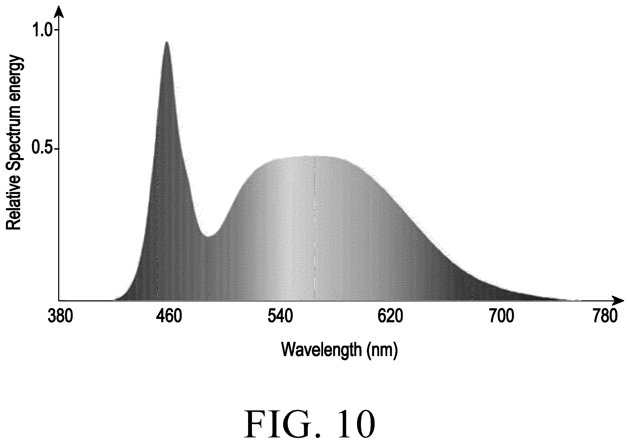

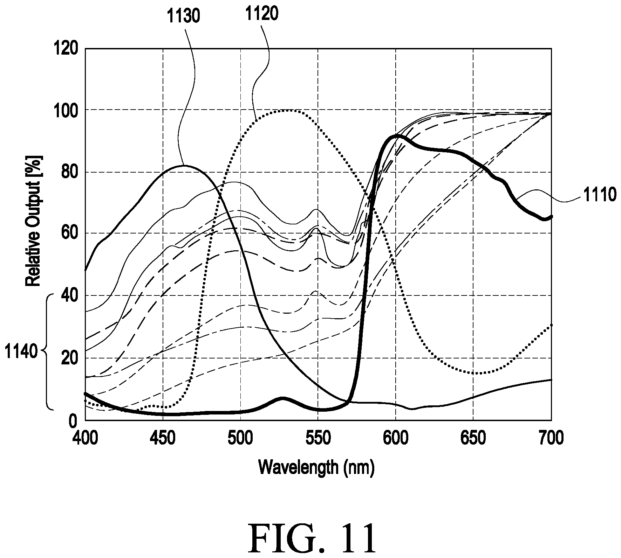

FIG. 9 is a block diagram illustrating a state in which light of a second wavelength band is irradiated by an electronic device according to various embodiments, FIG. 10 is a wavelength-intensity graph of light of a second wavelength band according to various embodiments, and FIG. 11 is a wavelength-intensity graph of light corresponding to at least one wavelength band included in a second wavelength band and RGB sensor reacting light according to various embodiments.

An electronic device according to various embodiments may operate a second light source to irradiate light of a second wavelength band corresponding to visible light to the skin. Since the skin pigment (melanin) may affect the amount of autofluorescence, the reflectance of the skin may be identified using the irradiation of light of the second wavelength band and the intensity of light corresponding to at least one wavelength band.

According to various embodiments, the second wavelength band may be the entire wavelength band of visible light having a specific color temperature, or a partial wavelength band of visible light having a specific color. In an embodiment, as illustrated in FIG. 10, the second wavelength band may be a white wavelength band evenly distributed over the entire wavelength band of visible light. For example, the second wavelength band may be white light having a color temperature of 5500 [K] or white light having high color rendition.

In another embodiment, the second wavelength band may be a partial wavelength band of visible light having a specific color. For example, some wavelength bands of visible light may be areas having relatively long wavelengths among visible light so as to be less related to autofluorescence of the skin.

The electronic device according to various embodiments may receive the second intensity of light corresponding to at least one wavelength band included in the second wavelength band from the sensing circuit while the second light source operates. The electronic device according to an embodiment may identify the skin reflectance based on the second intensity of light corresponding to at least one wavelength band included in the second wavelength band measured by the sensing circuit. The skin reflectance is to measure the intensity of light reflected by melanin and/or hemoglobin other than the advanced glycation end products.

The electronic device according to various embodiments may identify skin reflectance based on the second intensity of at least one light measured by the RGB sensor.

Specifically, as illustrated in FIG. 11, the skin reflectance 1140 is significantly different for each human skin. In particular, when light of the second wavelength band corresponding to visible light is irradiated to the skin, at least one reflected light may be distributed over the red wavelength area 1110, the green wavelength area 1120, and the blue wavelength area 1130 in the R sensor, the G sensor, and the B sensor of the RGB sensor (the RGB sensor 131 of FIG. 1), and the distribution over the wavelength bands may be different for each person.

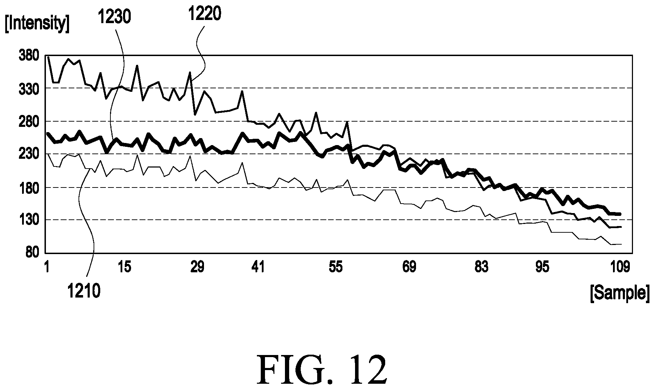

FIG. 12 is a graph of light intensity and skin reflectance in each wavelength area according to various embodiments. According to an embodiment, the electronic device may identify the skin reflectance based on the second intensity of at least one light included in the second wavelength band measured by the RGB sensor. The identification of skin reflectance is based on the purpose of estimating the distribution of melanin.

FIG. 12 illustrates an intensity of light measured by an RGB sensor in a state in which ambient light is blocked and white light 5500 [K] is irradiated to 110 types of pantone skin patches. In particular, the light intensity 1210 of the red wavelength area measured by the R sensor and the light intensity 1220 of the green wavelength area measured by the G sensor are illustrated, and the blue wavelength area is excluded because it is less related to skin reflectance. Further, the result 1230 of measuring the skin reflectance through an age reader for 110 types of pantone skin patches is illustrated. In comparison, the skin reflectance 1130 follows the light intensity 1210 of the red wavelength area for patches having relatively high skin reflectance (a strong intensity of reflected light), and follows the light intensity 1220 of the green wavelength area for patches having relatively low skin reflectance (a weak intensity of reflected light). Accordingly, the RGB skin reflectance model using the RGB sensor may be set to the following equation using the light intensity (Red) of the red wavelength area and the light intensity (Green) of the green wavelength area.

[Equation 1]

$$RGB \, Skin \, Reflectance \, Model = \alpha_1 * Red + \alpha_2 * (Green - Red) + \beta$$

Here, $\alpha_1$, $\alpha_2$, and $\beta$ may be changed depending on the lighting environment and may be estimated through regression analysis.

The electronic device according to various embodiments may receive, from the sensing circuit, at least one of the intensity of light of the green wavelength band or the intensity of light of the red wavelength band as the second intensity of at least one light. Since the blue wavelength band is less related to the skin reflectance, the electronic device according to an embodiment may identify the skin reflectance using only the intensity of the light of the green wavelength band and the intensity of the light of the red wavelength band.

The electronic device according to another embodiment may identify the skin reflectance using all of the intensity of the light of the green wavelength band, the intensity of the light of the red wavelength band, and the intensity of the light of the blue wavelength band.

The electronic device according to another embodiment may identify skin reflectance to estimate the distribution of melanin. In an embodiment, it may be converted into CIELAB using output data of the RGB sensor. The measurement value of the RGB sensor may be converted into a Lab value using the RGB2LAB function of the Matlab, and the coefficients of L, A, and B may be estimated using a correlation between the Lab value and the melanin distribution, thereby estimating the melanin distribution through the measurement value of the RGB sensor.

The electronic device according to still another embodiment may identify a melanin index (MI) and an erythema index (EI) using light intensity of a preset wavelength. The preset wavelength may be set to a wavelength with a small deviation between a person and a situation, and may be set to, e.g., 568 [nm], 660 [nm], and 890 [nm]. For example, the melanin index MI and the erythema index EI may be identified by the following equation using the intensity R of light.

[Equation 2]

$$MI = \log_{10}(R_{905nm} / R_{632nm}) \times 1000.$$
$$EI = \log_{10}(R_{632nm} / R_{540nm}) \times 1000.$$

In another embodiment, the distribution of melanin may be estimated using, e.g., measurement values of a plurality of wavelengths or an inclination value between the plurality of wavelengths preset in the melanin reflection characteristic graph.

The electronic device according to various embodiments may identify the skin reflectance based on the intensity of light measured by the RGB sensor or the identified melanin index or distribution. According to an embodiment, the electronic device may identify the skin reflectance using the intensity of the light measured by the RGB sensor relative to the intensity of the light of the second wavelength band irradiated from the second light source. In another embodiment, the electronic device may identify the skin reflectance according to the identified melanin index or melanin distribution, which may follow the relationship of the skin reflectance matched according to the previously stored melanin index or melanin distribution.

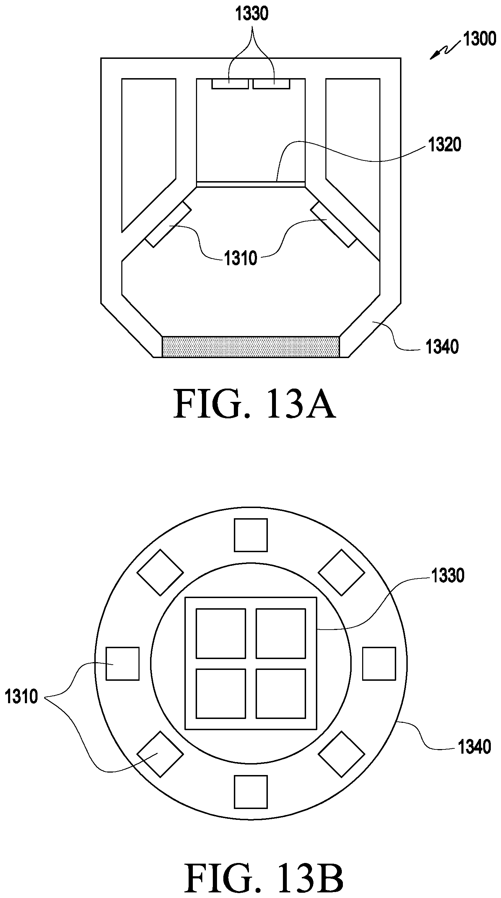

FIG. 13A is a cross-sectional view illustrating an electronic device 1300 according to various embodiments, and FIG. 13B is a bottom view illustrating an electronic device 1300 according to various embodiments. The electronic device 1300 according to various embodiments may be formed in various structures to obtain an optimal result, and the configuration illustrated in FIGS. 13A and 13B is illustrated as an example.

An electronic device 1300 according to various embodiments may include a plurality of light sources 1310, a polarizing filter 1320, a photodiode 1330, and a light blocking housing 1340.

Each of the plurality of light sources 1310 may be configured to irradiate light of a different wavelength band to the skin. In an embodiment, the plurality of light sources 1310 may be disposed to irradiate light toward the skin, but may be disposed to be inclined obliquely to minimize specular reflection. The plurality of light sources 1310 may be configured to irradiate light of a wavelength band (e.g., a first wavelength band) corresponding to UV light or a wavelength band (e.g., a second wavelength band) corresponding to visible light, or may be configured to irradiate light of a wavelength band corresponding to IR light. Alternatively, at least some of the plurality of light sources 1310 may be sub-color light sources such as an R light source, a G light source, or a B light source, and those skilled in the art will understand that the sub-color light source may be driven to irradiate visible light (e.g., white light) when irradiating visible light.

The polarizing filter 1320 may block at least some light output from the plurality of light sources 1310 and reflected, and may be disposed in front of the photodiode 1330.

A plurality of photodiodes 1330 may be provided to sense light intensities of R, G, and B bands, respectively, so as to measure the light intensity of the entire visible wavelength band. For example, a plurality of photodiodes 1330 which respectively measure the light intensities of R, G, B, and W bands or the light intensities of R, G, G, and B bands may be provided.

The light blocking housing 1340 may be formed so that one surface of the light blocking housing 1340, which is in tight contact with the skin, is open or transparent to transmit light, and the periphery surrounding the one surface may be formed of an opaque material to prevent transmission of light.

FIG. 14 illustrates an application example of an electronic device 1400 according to an embodiment. The electronic device 1400 according to an embodiment may be configured to be in tight contact with the user's skin in a wearable type. For example, the electronic device 1400 may be of a watch type in which the lower surface is in tight contact with the user's wrist.

The watch-type electronic device 1400 according to an embodiment may include a first light source 1410 and a second light source 1430 disposed on a lower surface thereof to irradiate light to the user's wrist skin, and may further include a photodiode 1430 for measuring the intensity of reflected or fluorescent light according to the light irradiated from the first light source 1410 and the second light source 1420.

The watch-type electronic device 1400 according to an embodiment may identify the advanced glycation end products data of the skin based on the intensity of light measured through the photodiode 1430, may directly display the identified advanced glycation end products data of the skin on a display (not shown), or may transmit the identified advanced glycation end products data to an external device (e.g., a mobile phone) connected through communication.

FIG. 15 illustrates an application example of an electronic device 1520 according to other embodiments. The electronic device 1520 according to another embodiment may be of an accessory type mounted on an external device 1510 (e.g., a mobile phone or the electronic device 1601 of FIG. 16) connected through communication. The accessory-type electronic device 1520 according to another embodiment may operate in conjunction with the external device 1510 while communicating with the external device 1510, and may supply power by itself or may receive power from the external device 1510.

The accessory-type electronic device 1520 according to another embodiment may include a light source (not shown) irradiating light of a first wavelength band corresponding to UV light and light of a second wavelength band corresponding to visible light to the skin, respectively, and a communication circuit (e.g., the communication circuit 160 of FIG. 1) communicating with the external device 1510. The accessory-type electronic device 1520 according to another embodiment may be connected to the external device 1510 (e.g., a mobile phone or the electronic device 1601 of FIG. 16) through communication, and may utilize components included in the external device 1510. For example, the accessory-type electronic device 1520 may utilize the configuration of the external device 1510 (e.g., a mobile phone) without including the configuration of the sensing circuit or the processor.

The external device 1510 (e.g., a mobile phone or the electronic device 1601 of FIG. 16) connected to the accessory-type electronic device 1520 according to another embodiment via communication may include a sensing circuit (e.g., the sensing circuit 130 of FIG. 1, the RGB sensor 131, or the sensor circuit 1676 of FIG. 16) measuring the intensity of light incident from the skin by reflection or fluorescence and outputting the measured intensity of light and a processor (e.g., the processor 1620 of FIG. 16).

The external device 1510 (e.g., the electronic device 1601 of FIG. 16) according to various embodiments may communicate with the accessory-type electronic device 1520 through a communication circuit (e.g., the communication module 1690 of FIG. 16), and may receive a state in which light is irradiated from the accessory-type electronic device 1520 through communication. The communication circuit (e.g., the communication module 1690 of FIG. 16) of the external device 1510 (e.g., the electronic device 1601 of FIG. 16) may be configured to output, to the processor (e.g., the processor 1620 of FIG. 16), the state in which light of the first wavelength band is irradiated or the state in which light of the second wavelength band is irradiated, and the processor (e.g., the processor 1620 of FIG. 16) may receive the first intensity of light and the second intensity of at least one light from the sensing circuit (e.g., the sensing circuit 130 of FIG. 1 or the RGB sensor 131) based on the received state in which the light of the first wavelength band is irradiated or state in which the light of the second wavelength band is irradiated.

FIG. 16 is a block diagram illustrating an electronic device 1601 in a network environment according to various embodiments. An electronic device 1601 (e.g., the electronic device 100 of FIG. 1, the electronic device 1400 of FIG. 14, or the external device 1510 of FIG. 15) in the network environment 1601 may communicate with an electronic device 1602 via a first network 1698 (e.g., a short-range wireless communication network), or an electronic device 1604 or a server 1608 via a second network 1699 (e.g., a long-range wireless communication network). According to an embodiment, the electronic device 1601 may communicate with the electronic device 1604 via the server 1608. According to an embodiment, the electronic device 1601 may include a processor 1620, memory 1630, an input module 1650, a sound output module 1655, a display module 1660, an audio module 1670, a sensor circuit 1676, an interface 1677, a connecting terminal 1678, a haptic module 1679, a camera module 1680, a power management module 1688, a battery 1689, a communication module 1690, a subscriber identification module (SIM) 1696, or an antenna module 1697. In an embodiment, at least one (e.g., the connecting terminal 1678) of the components may be omitted from the electronic device 1601, or one or more other components may be added in the electronic device 101. According to an embodiment, some (e.g., the sensor circuit 1676, the camera module 1680, or the antenna module 1697) of the components may be integrated into a single component (e.g., the display module 1660).

The processor 1620 may execute, for example, software (e.g., a program 1640) to control at least one other component (e.g., a hardware or software component) of the electronic device 1601 coupled with the processor 1620, and may perform various data processing or computation. According to an embodiment, as at least part of the data processing or computation, the processor 1620 may store a command or data received from another component (e.g., the sensor circuit 1676 or the communication module 1690) in volatile memory 1632, process the command or the data stored in the volatile memory 1632, and store resulting data in non-volatile memory 1634. According to an embodiment, the processor 1620 may include a main processor 1621 (e.g., a central processing unit (CPU) or an application processor (AP)), or an auxiliary processor 1623 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 1601 includes the main processor 1621 and the auxiliary processor 1623, the auxiliary processor 1623 may be configured to use lower power than the main processor 1621 or to be specified for a designated function. The auxiliary processor 1623 may be implemented as separate from, or as part of the main processor 1621.

The auxiliary processor 1623 may control at least some of functions or states related to at least one component (e.g., the display module 1660, the sensor circuit 1676, or the communication module 1690) among the components of the electronic device 1601, instead of the main processor 1621 while the main processor 1621 is in an inactive (e.g., sleep) state, or together with the main processor 1621 while the main processor 1621 is in an active state (e.g., executing an application). According to an embodiment, the auxiliary processor 1623 (e.g., an image signal processor or a communication processor) may be implemented as part of another component (e.g., the camera module 1680 or the communication module 1690) functionally related to the auxiliary processor 123. According to an embodiment, the auxiliary processor 1623 (e.g., the neural processing unit) may include a hardware structure specified for artificial intelligence model processing. The artificial intelligence model may be generated via machine learning. Such learning may be performed, e.g., by the electronic device 1601 where the artificial intelligence is performed or via a separate server (e.g., the server 1608). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 1630 may store various data used by at least one component (e.g., the processor 1620 or the sensor circuit 1676) of the electronic device 1601. The various data may include, for example, software (e.g., the program 1640) and input data or output data for a command related thereto. The memory 1630 may include the volatile memory 1632 or the non-volatile memory 1634.

The program 1640 may be stored in the memory 1630 as software, and may include, for example, an operating system (OS) 1642, middleware 1644, or an application 1646.

The input module 1650 may receive a command or data to be used by other component (e.g., the processor 1620) of the electronic device 1601, from the outside (e.g., a user) of the electronic device 1601. The input module 1650 may include, for example, a microphone, a mouse, a keyboard, keys (e.g., buttons), or a digital pen (e.g., a stylus pen).

The sound output module 1655 may output sound signals to the outside of the electronic device 1601. The sound output module 1655 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 1660 may visually provide information to the outside (e.g., a user) of the electronic device 1601. The display 1660 may include, for example, a display, a hologram device, or a projector and control circuitry to control a corresponding one of the display, hologram device, and projector. According to an embodiment, the display 1660 may include a touch sensor configured to detect a touch, or a pressure sensor configured to measure the intensity of a force generated by the touch.

The audio module 1670 may convert a sound into an electrical signal and vice versa. According to an embodiment, the audio module 1670 may obtain the sound via the input module 1650, or output the sound via the sound output module 1655 or a headphone of an external electronic device (e.g., an electronic device 1602) directly (e.g., wiredly) or wirelessly coupled with the electronic device 1601.

The sensor circuit 1676 may detect an operational state (e.g., power or temperature) of the electronic device 1601 or an environmental state (e.g., a state of a user) external to the electronic device 101, and then generate an electrical signal or data value corresponding to the detected state. According to an embodiment, the sensor circuit 1676 may include, for example, a gesture sensor, a gyro sensor, an atmospheric pressure sensor, a magnetic sensor, an accelerometer, a grip sensor, a proximity sensor, a color sensor, an infrared (IR) sensor, a biometric sensor, a temperature sensor, a humidity sensor, or an illuminance sensor.

The interface 1677 may support one or more specified protocols to be used for the electronic device 1601 to be coupled with the external electronic device (e.g., the electronic device 1602) directly (e.g., wiredly) or wirelessly. According to an embodiment, the interface 1677 may include, for example, a high definition multimedia interface (HDMI), a universal serial bus (USB) interface, a secure digital (SD) card interface, or an audio interface.

A connecting terminal 1678 may include a connector via which the electronic device 1601 may be physically connected with the external electronic device (e.g., the electronic device 1602). According to an embodiment, the connecting terminal 1678 may include, for example, a HDMI connector, a USB connector, a SD card connector, or an audio connector (e.g., a headphone connector).

The haptic module 1679 may convert an electrical signal into a mechanical stimulus (e.g., a vibration or motion) or electrical stimulus which may be recognized by a user via his tactile sensation or kinesthetic sensation. According to an embodiment, the haptic module 1679 may include, for example, a motor, a piezoelectric element, or an electric stimulator.

The camera module 1680 may capture a still image or moving images. According to an embodiment, the camera module 1680 may include one or more lenses, image sensors, image signal processors, or flashes.

The power management module 1688 may manage power supplied to the electronic device 1601. According to an embodiment, the power management module 1688 may be implemented as at least part of, for example, a power management integrated circuit (PMIC).

The battery 1689 may supply power to at least one component of the electronic device 1601. According to an embodiment, the battery 1689 may include, for example, a primary cell which is not rechargeable, a secondary cell which is rechargeable, or a fuel cell.

The communication module 1690 may support establishing a direct (e.g., wired) communication channel or a wireless communication channel between the electronic device 1601 and the external electronic device (e.g., the electronic device 1602, the electronic device 1604, or the server 1608) and performing communication via the established communication channel. The communication module 1690 may include one or more communication processors that are operable independently from the processor 1620 (e.g., the application processor (AP)) and supports a direct (e.g., wired) communication or a wireless communication. According to an embodiment, the communication module 1690 may include a wireless communication module 1692 (e.g., a cellular communication module, a short-range wireless communication module, or a global navigation satellite system (GNSS) communication module) or a wired communication module 1694 (e.g., a local area network (LAN) communication module or a power line communication (PLC) module). A corresponding one of these communication modules may communicate with the external electronic device 1604 via a first network 1698 (e.g., a short-range communication network, such as BluetoothTM, wireless-fidelity (Wi-Fi) direct, or infrared data association (IrDA)) or a second network 1699 (e.g., a long-range communication network, such as a legacy cellular network, a 5G network, a next-generation communication network, the Internet, or a computer network (e.g., local area network (LAN) or wide area network (WAN)). These various types of communication modules may be implemented as a single component (e.g., a single chip), or may be implemented as multi components (e.g., multi chips) separate from each other. The wireless communication module 1692 may identify or authenticate the electronic device 1601 in a communication network, such as the first network 1698 or the second network 1699, using subscriber information (e.g., international mobile subscriber identity (IMSI)) stored in the subscriber identification module 1696.

The wireless communication module 1692 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 1692 may support a high-frequency band (e.g., the mmWave band) to achieve, e.g., a high data transmission rate. The wireless communication module 1692 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 1692 may support various requirements specified in the electronic device 1601, an external electronic device (e.g., the electronic device 1604), or a network system (e.g., the second network 1699). According to an embodiment, the wireless communication module 1692 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 1697 may transmit or receive a signal or power to or from the outside (e.g., the external electronic device). According to an embodiment, the antenna module 1697 may include one antenna including a radiator formed of a conductor or conductive pattern formed on a substrate (e.g., a printed circuit board (PCB)). According to an embodiment, the antenna module 1697 may include a plurality of antennas (e.g., an antenna array). In this case, at least one antenna appropriate for a communication scheme used in a communication network, such as the first network 1698 or the second network 1699, may be selected from the plurality of antennas by, e.g., the communication module 1690. The signal or the power may then be transmitted or received between the communication module 1690 and the external electronic device via the selected at least one antenna. According to an embodiment, other parts (e.g., radio frequency integrated circuit (RFIC)) than the radiator may be further formed as part of the antenna module 1697.

According to various embodiments, the antenna module 1697 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, instructions or data may be transmitted or received between the electronic device 1601 and the external electronic device 1604 via the server 1608 coupled with the second network 1699. The external electronic devices 1602 or 1104 each may be a device of the same or a different type from the electronic device 1601. According to an embodiment, all or some of operations to be executed at the electronic device 1601 may be executed at one or more of the external electronic devices 1602, 1104, or 1108. For example, if the electronic device 1601 should perform a function or a service automatically, or in response to a request from a user or another device, the electronic device 1601, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 1601. The electronic device 1601 may provide the outcome, with or without further processing of the outcome, as at least part of a reply to the request. To that end, a cloud computing, distributed computing, mobile edge computing (MEC), or client-server computing technology may be used, for example. The electronic device 1601 may provide ultra low-latency services using, e.g., distributed computing or mobile edge computing. In another embodiment, the external electronic device 1604 may include an Internet-of-things (IoT) device. The server 1608 may be an intelligent server using machine learning and/or a neural network. According to an embodiment, the external electronic device 1604 or the server 1608 may be included in the second network 1699. The electronic device 1601 may be applied to intelligent services (e.g., smart home, smart city, smart car, or healthcare) based on 5G communication technology or IoT-related technology.

For example, examples of the electronic device according to embodiments of the present disclosure may include at least one of a smartphone, a tablet personal computer (PC), a mobile phone, a video phone, an e-book reader, a desktop PC, a laptop computer, a netbook computer, a workstation, a PDA (personal digital assistant), a portable multimedia player (PMP), an MP3 player, a mobile medical device, a camera, or a wearable device. The wearable device may include at least one of an accessory-type device (e.g., a watch, a ring, a bracelet, an anklet, a necklace, glasses, contact lenses, or a head-mounted device (HMD)), a fabric- or clothes-integrated device (e.g., electronic clothes), a body attaching-type device (e.g., a skin pad or tattoo), or a body implantable device. In some embodiments, examples of the smart home appliance may include at least one of a television, a digital video disk (DVD) player, an audio player, a refrigerator, an air conditioner, a cleaner, an oven, a microwave oven, a washer, a drier, an air cleaner, a set-top box, a home automation control panel, a security control panel, a TV box (e.g., Samsung HomeSync™, Apple TV™, or Google TV™), a gaming console (Xbox™, PlayStation™), an electronic dictionary, an electronic key, a camcorder, or an electronic picture frame.

According to an embodiment of the present disclosure, examples of the electronic device may include at least one of various medical devices (e.g., diverse portable medical measuring devices (a blood sugar measuring device, a heartbeat measuring device, or a body temperature measuring device), a magnetic resource angiography (MRA) device, a magnetic resource imaging (MRI) device, a computed tomography (CT) device, an imaging device, or an ultrasonic device), a navigation device, a global navigation satellite system (GNSS) receiver, an event data recorder (EDR), a flight data recorder (FDR), an automotive infotainment device, an sailing electronic device (e.g., a sailing navigation device or a gyro compass), avionics, security devices, vehicular head units, industrial or home robots, drones, automatic teller's machines (ATMs) of financial organizations, point of sales (POS) devices of stores, or Internet of things devices (e.g., a bulb, various sensors, a sprinkler, a fire alarm, a thermostat, a street light, a toaster, fitness equipment, a hot water tank, a heater, or a boiler). According to various embodiments of the disclosure, examples of the electronic device may at least one of part of furniture or building/structure, an electronic board, an electronic signature receiving device, a projector, or various measurement devices (e.g., devices for measuring water, electricity, gas, or electromagnetic waves). According to embodiments of the present invention, the electronic device may be flexible or may be a combination of the above-enumerated electronic devices. According to an embodiment of the disclosure, the electronic devices are not limited to those described above. As used herein, the term "user" may denote a human or another device (e.g., an artificial intelligent electronic device) using the electronic device.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used herein, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 1640) including one or more instructions that are stored in a storage medium (e.g., internal memory 1636 or external memory 1638) that is readable by a machine (e.g., the electronic device 1601). For example, a processor (e.g., the processor 1620) of the machine (e.g., the electronic device 1601) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The storage medium readable by the machine may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program products may be traded as commodities between sellers and buyers. The computer program product may be distributed in the form of a machine-readable storage medium (e.g., compact disc read only memory (CD-ROM)), or be distributed (e.g., downloaded or uploaded) online via an application store (e.g., Play StoreTM), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities. Some of the plurality of entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

**Claims**

1. An electronic device, comprising:

a first light source configured to irradiate light of a first wavelength band corresponding to UV light;
a second light source configured to irradiate light of a second wavelength band corresponding to visible light, the second wavelength band at least partially different from the first wavelength band;
a sensing circuit configured to measure an intensity of incident light and output the measured intensity of light; and
a processor configured to:

receive, from the sensing circuit, a first intensity of light corresponding to a third wavelength band, at least partially different from the first wavelength band, measured by the sensing circuit while the first light source operates;
receive, from the sensing circuit, a second intensity of light respectively corresponding to at least one wavelength band included in the second wavelength band measured by the sensing circuit while the second light source operates; and
provide advanced glycation end products (AGEs) data of skin based on the received first intensity of light and the received second intensity of at least one light.

2. The electronic device of claim 1, wherein the first wavelength band is a wavelength band corresponding to a wavelength band of UV-A light.

3. The electronic device of claim 1, wherein the second wavelength band is an entire wavelength band of visible light having a specific color temperature or a partial wavelength band of visible light having a specific color.

4. The electronic device of claim 1, wherein the third wavelength band is a partial wavelength band of visible light set to be larger than a first boundary wavelength between visible light and UV and set to be smaller than a second boundary wavelength between visible light and infrared light.

5. The electronic device of claim 1, wherein the sensing circuit is configured to include an RGB sensor configured to measure an intensity of incident light, and wherein the processor is configured to receive an intensity of light corresponding to a green wavelength band as the first intensity of the light from the sensing circuit while the first light source operates.

6. The electronic device of claim 1, wherein the detection circuit is configured to include an RGB sensor configured to measure an intensity of incident light, and wherein the processor is configured to receive at least one of an intensity of light corresponding to a green wavelength band or an intensity of light of a red wavelength band as the second intensity of the at least one light from the sensing circuit while the second light source operates.

7. The electronic device of claim 1, wherein the processor is configured to identify a skin reflectance based on the received second intensity of the at least one light and provide advanced glycation end products (AGEs) data of skin based on the received first intensity of light and the identified skin reflectance.

8. The electronic device of claim 1, wherein the sensing circuit is configured to include an RGB sensor configured to measure an intensity of incident light, and wherein the sensing circuit is configured to output at least one of an intensity of light of a blue wavelength band, an intensity of light of a green wavelength band, or an intensity of light of a red wavelength band, measured by the RGB sensor, in a state of raw data that is not subjected to image processing.

9. The electronic device of claim 1, wherein the sensing circuit is configured to include a plurality of pixels configured to measure each of intensity of light incident on a plurality of areas, and wherein the sensing circuit is configured to output a representative value set based on the intensity of light measured by each of the plurality of pixels.

10. A method for operating an electronic device, the method comprising:

receiving, from a sensing circuit, a first intensity of light corresponding to a third wavelength band at least partially different from a first wavelength band measured by the sensing circuit while a first light source configured to irradiate light of the first wavelength band corresponding to UV light operates and a second intensity of light corresponding to at least one wavelength band included in a second wavelength band measured by the sensing circuit while a second light source configured to irradiate light of the second wavelength band corresponding to visible light operates; and
providing advanced glycation end products (AGEs) of skin based on the received first intensity of light and second intensity of at least one light.

11. The method of claim 10, wherein the sensing circuit is configured to include an RGB sensor configured to measure an intensity of incident light, and wherein receiving the received first intensity of light and second intensity of the at least one light receives an intensity of light corresponding to a green wavelength band as the first intensity of the light from the sensing circuit while the first light source operates.

12. The method of claim 10, wherein the sensing circuit is configured to include an RGB sensor configured to measure an intensity of incident light, and wherein receiving the received first intensity of light and second intensity of the at least one light receives at least one of an intensity of light corresponding to a green wavelength band or an intensity of light of a red wavelength band as the second intensity of the at least one light from the sensing circuit while the second light source operates.

13. The method of claim 10, further comprising identifying a skin reflectance based on the received second intensity of the at least one light, wherein providing the skin advanced glycation end products provides advanced glycation end products (AGEs) data of skin based on the received first intensity of light and the identified skin reflectance.

14. The method of claim 10, wherein the sensing circuit is configured to include an RGB sensor configured to measure an intensity of incident light, and wherein receiving the received first intensity of light and second intensity of the at least one light receives at least one of an intensity of light of a blue wavelength band, an intensity of light of a green wavelength band, or an intensity of light of a red wavelength band, measured by the RGB sensor, in a state of raw data that is not subjected to image processing.

15. The method of claim 10, wherein the sensing circuit is configured to include a plurality of pixels configured to measure each of intensity of light incident on a plurality of areas, and wherein receiving the received first intensity of light and second intensity of the at least one light receives a representative value set based on an intensity of light measured by each of the plurality of pixels.

100

150

Display

140

Processor

160

Communication circuit

~170

Sensing circuit

~130

131

R Sensor

131

G Sensor

131

B Sensor

First light source

110

Driving module

180

Second light source

120

FIG. 1

START

↓

RECEIVE FIRST INTENSITY OF LIGHT CORRESPONDING TO
THIRD WAVELENGTH BAND WHILE FIRST LIGHT SOURCE OPERATES
AND SECOND INTENSITY OF LIGHT CORRESPONDING TO AT LEAST
ONE WAVELENGTH BAND INCLUDED IN SECOND WAVELENGTH
BAND WHILE SECOND LIGHT SOURCE OPERATES — 210

↓

IDENTIFY SKIN REFLECTANCE BASED ON
SECOND INTENSITY OF AT LEAST ONE LIGHT — 220

↓

PROVIDE SKIN ADVANCED GLYCATION END PRODUCTS DATA
BASED ON FIRST INTENSITY OF LIGHT AND SKIN REFLECTANCE — 230

↓

END

200

FIG. 2

FIG. 3

| Molecule | Excitation (nm) | Fluorescence (nm) Peak | Organisms |
|---|---|---|---|
| NAD(P)H | 340 | 450 | All |
| Chlorophyll | 465 / 665 | 673 / 726 | Plants |
| Collagen | 270-370 | 305-450 | Animals |
| Retinol | | 500 | Animals & bacteria |
| Riboflavin | | 550 | All |
| Cholecalciferol | | 380-460 | Animals |
| Folic acid | | 450 | All |
| Pyridoxine | | 400 | All |
| Tyrosine | 270 | 305 | All |
| Dityrosine | 325 | 400 | Animals |
| Excimer-like affregate | 270 | 360 | Animals |
| Glycation adduct | 370 | 450 | Animals |
| Indolamine | | | Animals |
| Lipofuscin | 410-470 | 500-695 | Eukaryotes |
| Lignin, a polyphenol | 335 / 488 | 455 / 535 | Plants |
| Tryptophan | 280 | 300-350 | All |
| Flavin | 380-490 | 520-560 | All |
| Melanin | 340-400 | 360-560 | Animals |

FIG. 4

500

510                                                        530

**Light of first wavelength band** ~511

**Measure skin fluorescence** 513

**First intensity of third wavelength band (green value)** 515

**Advanced glycation end products data** 533

**Light of second wavelength band** ~521

**Measure skin reflectance** 523

**Second intensity of at least one wavelength band (green, red values) included in third wavelength band** 525

**Identify skin reflectance** 531

520

# FIG. 5

FIG. 6

FIG. 7

FIG. 8

## FIG. 9

FIG. 10

FIG. 11

FIG. 12

FIG. 13A

FIG. 13B

# FIG. 14

# FIG. 15

FIG. 16

EP 4 467 075 A1

<div align="center">

**INTERNATIONAL SEARCH REPORT**

</div>

| International application No. |
|---|
| **PCT/KR2022/021494** |

**A.  CLASSIFICATION OF SUBJECT MATTER**

**A61B 5/1455**(2006.01)i; **A61B 5/145**(2006.01)i; **A61B 5/00**(2006.01)i

According to International Patent Classification (IPC) or to both national classification and IPC

**B.  FIELDS SEARCHED**

Minimum documentation searched (classification system followed by classification symbols)

A61B 5/1455(2006.01); A61B 5/00(2006.01); A61B 5/024(2006.01); A61B 5/145(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 최종당화산물(AGEs: Advanced Glycation End products), 웨어러블(wearable), 파장(wavelength), 디텍터(detector)

**C.  DOCUMENTS CONSIDERED TO BE RELEVANT**

| Category* | Citation of document, with indication, where appropriate, of the relevant passages | Relevant to claim No. |
|---|---|---|
| X | (주)필로시스 등. 광학기반 무채혈 당대사이상 모니터링을 위한 웨어러블 장비개발. 산업통상자원부, 기술개발사업 최종보고서. pp. 1-98, 15 February 2021, non-official translation (PHILOSYS CO., LTD. et al. Development of Wearable Devices for Optical-Based Non-Blood Glucose Metabolism Monitoring. Final Report of Technology Development Project, Ministry of Trade, Industry and Energy).<br>  [Retrieved on 02 March 2023]. Retrieved from <https://scienceon.kisti.re.kr/commons/util/originalView.do?cn=TRKO202200005916&dbt=TRKO&rn=>.<br>  See pages 50, 59-60 and 62-64; and figures 58-62 and 66-68. | 1-15 |
| A | KR 10-2348195 B1 (VARYSEN) 07 January 2022 (2022-01-07)<br>  See claims 1-5 and 7-9; and figures 1-17. | 1-15 |
| A | KR 10-2020-0113997 A (PHILOSYS CO., LTD.) 07 October 2020 (2020-10-07)<br>  See paragraphs [0035]-[0099]; claims 1-14; and figures 1-6. | 1-15 |
| A | KR 10-2020-0044409 A (SAMSUNG ELECTRONICS CO., LTD.) 29 April 2020 (2020-04-29)<br>  See claims 1-30; and figures 1a-10. | 1-15 |

☑ Further documents are listed in the continuation of Box C.          ☑ See patent family annex.

| \* | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **04 April 2023** | **04 April 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

<div align="center">

**32**

</div>

**EP 4 467 075 A1**

<table>
<tr><td colspan="3"><b>INTERNATIONAL SEARCH REPORT</b></td><td>International application No.<br><b>PCT/KR2022/021494</b></td></tr>
<tr><td colspan="4"><b>C.     DOCUMENTS CONSIDERED TO BE RELEVANT</b></td></tr>
<tr><td>Category*</td><td colspan="2">Citation of document, with indication, where appropriate, of the relevant passages</td><td>Relevant to claim No.</td></tr>
<tr><td>A</td><td colspan="2">KR 10-2020-0067124 A (DEXCOM, INC.) 11 June 2020 (2020-06-11)<br>See paragraphs [0074]-[0094]; and claims 1-190.</td><td>1-15</td></tr>
</table>

Form PCT/ISA/210 (second sheet) (July 2022)

International application No.

**PCT/KR2022/021494**

| Patent document cited in search report | | | Publication date (day/month/year) | Patent family member(s) | | | Publication date (day/month/year) |
|---|---|---|---|---|---|---|---|
| KR | 10-2348195 | B1 | 07 January 2022 | | None | | |
| KR | 10-2020-0113997 | A | 07 October 2020 | KR | 10-2020-0113998 | A | 07 October 2020 |
| | | | | US | 2022-0192542 | A1 | 23 June 2022 |
| | | | | WO | 2020-197276 | A1 | 01 October 2020 |
| KR | 10-2020-0044409 | A | 29 April 2020 | CN | 111067502 | A | 28 April 2020 |
| | | | | EP | 3639732 | A1 | 22 April 2020 |
| | | | | EP | 3639732 | B1 | 30 June 2021 |
| | | | | US | 11490863 | B2 | 08 November 2022 |
| | | | | US | 2020-0121259 | A1 | 23 April 2020 |
| KR | 10-2020-0067124 | A | 11 June 2020 | AU | 2018-295116 | A1 | 12 December 2019 |
| | | | | AU | 2018-295116 | B2 | 15 July 2021 |
| | | | | AU | 2021-245266 | A1 | 04 November 2021 |
| | | | | CN | 110996775 | A | 10 April 2020 |
| | | | | EP | 3641636 | A1 | 29 April 2020 |
| | | | | EP | 3641636 | A4 | 17 March 2021 |
| | | | | EP | 3641636 | B1 | 10 November 2021 |
| | | | | EP | 3928688 | B1 | 01 June 2022 |
| | | | | EP | 4008240 | A1 | 08 June 2022 |
| | | | | EP | 4111949 | A1 | 04 January 2023 |
| | | | | JP | 2020-525101 | A | 27 August 2020 |
| | | | | MX | 2019015365 | A | 20 July 2020 |
| | | | | US | 10863944 | B2 | 15 December 2020 |
| | | | | US | 10905377 | B2 | 02 February 2021 |
| | | | | US | 11134896 | B2 | 05 October 2021 |
| | | | | US | 11207026 | B2 | 28 December 2021 |
| | | | | US | 11253201 | B2 | 22 February 2022 |
| | | | | US | 11311240 | B2 | 26 April 2022 |
| | | | | US | 11311241 | B2 | 26 April 2022 |
| | | | | US | 11395631 | B2 | 26 July 2022 |
| | | | | US | 11504063 | B2 | 22 November 2022 |
| | | | | US | 11510625 | B2 | 29 November 2022 |
| | | | | US | 2018-0368771 | A1 | 27 December 2018 |
| | | | | US | 2018-0368772 | A1 | 27 December 2018 |
| | | | | US | 2018-0368773 | A1 | 27 December 2018 |
| | | | | US | 2018-0368774 | A1 | 27 December 2018 |
| | | | | US | 2021-0052224 | A1 | 25 February 2021 |
| | | | | US | 2021-0219918 | A1 | 22 July 2021 |
| | | | | US | 2021-0219919 | A1 | 22 July 2021 |
| | | | | US | 2021-0307693 | A1 | 07 October 2021 |
| | | | | US | 2021-0307694 | A1 | 07 October 2021 |
| | | | | US | 2021-0307695 | A1 | 07 October 2021 |
| | | | | US | 2021-0338160 | A1 | 04 November 2021 |
| | | | | US | 2021-0338161 | A1 | 04 November 2021 |
| | | | | US | 2021-0338162 | A1 | 04 November 2021 |
| | | | | US | 2022-0151562 | A1 | 19 May 2022 |
| | | | | US | 2022-0151563 | A1 | 19 May 2022 |
| | | | | US | 2022-0361820 | A1 | 17 November 2022 |
| | | | | WO | 2019-005627 | A1 | 03 January 2019 |

Form PCT/ISA/210 (patent family annex) (July 2022)